# EUROPEAN PATENT SPECIFICATION

(11) **EP 3 077 785 B1**
(45) Date of publication and mention of the grant of the patent: **06.07.2022**
(21) Application number: 14867573.9
(22) Date of filing: 05.12.2014
(51) Int. Cl.: G01N 1/20, G01N 33/28, G01N 1/10

(54) **METHOD FOR ANALYSIS OF A LIQUID WITH A SAMPLING UNIT**
VERFAHREN ZUR ANALYSE EINER FLÜSSIGKEIT MIT EINER PROBENAHMEEINHEIT D'UN
PROCÉDÉ D'ANALYSE D'UN LIQUIDE AVEC UNE UNITÉ D'ÉCHANTILLONAGE

(30) Priority: 06.12.2013 SE 1351455
(43) Date of publication of application: 12.10.2016
(73) Proprietor: Scania CV AB, 151 87 Södertälje (SE)
(72) Inventor: EURENIUS, Klas, 151 46 Södertälje (SE); ERIKSSON, Henrik, 141 59 Huddinge (SE)
(74) Representative: Scania CV AB
(86) International application number: PCT/SE2014/051458
(87) International publication number: WO 2015/084251

(56) References cited:
- WO-A1-93/16365
- WO-A1-2013/002713
- WO-A1-2013/002713
- WO-A1-2014/074054
- JP-A- H1 123 429
- US-A1- 2004 063 215
- US-A1- 2004 173 035
- US-A1- 2009 211 379

## Description

### FIELD OF TECHNOLOGY

The invention relates to a method for sampling of a liquid with a sampling unit.

### BACKGROUND OF THE INVENTION AND PRIOR ART

Diesel powered motor vehicles are equipped with exhaust purification devices with the objective of reducing emissions of particles and chemicals occurring in the diesel engine's exhausts. There are also different standards and statutory requirements governing allowable exhaust emissions from vehicles. Prior art exhaust purification devices are sensitive to high levels of sulphur in the fuel. A level exceeding around 10 ppm sulphur in the fuel may lead to a poor reduction of emissions in the exhaust purification device. In order to reduce this risk and ensure that the legislative requirements are met, some vehicle manufacturers specify the maximum level of sulphur which the fuel may contain, for example that the level of sulphur must be less than 10 ppm. Fuel with higher sulphur levels are often cheaper and therefore more attractive to use in a vehicle. Such fuel damages vehicles, but it is difficult to determine in retrospect and to prove that the vehicle was fuelled with fuel having a sulphur level exceeding 10 ppm.

Other compounds as well, such as phosphorous compounds, may be damaging to the vehicle and, more importantly, to the environment. However, it is difficult, also in connection with these compounds, to determine in retrospect and to prove that the vehicle was fuelled with fuel having harmful concentrations of such unwanted chemicals or compounds.

In order to reduce emissions of particles and nitrogen oxides (NOx), exhaust treatment systems are used, which may comprise for example a diesel oxidation catalyst (DOC), a particulate filter and so-called NOx-reducers, for example an EGR (Exhaust Gas Reduction)-system and selective catalytic reduction (SCR), in the exhaust stream from combustion engines, for example in vehicles. The efficiency for such exhaust treatment systems and specifically diesel oxidation catalysts is reduced at the occurrence of compounds containing sulphur. Such sulphur-containing compounds (for example mercaptans, thiols, thiophenes, thioethers, thioesters, disulfides) and for example sulphur-containing aromatic compounds, "poison" or react with the diesel oxidation catalyst and/or other components of the exhaust treatment system and these parts of the system therefore have a reduced efficiency leading to corrosion problems in the engine and increased exhaust emissions. The diesel oxidation catalyst is sensitive to high levels of sulphur and may therefore have a shortened life at excessively high sulphur levels in the fuel. It is therefore important to be able to analyse whether the vehicle has been fuelled for a period of time with fuel having too high a sulphur level.

US-2002/0079236 relates to a sensor to measure the concentration of sulphur compounds in a liquid. The sensor comprises two electrodes, one active electrode, which is in contact with the liquid to be measured, and one reference electrode, which is isolated from the liquid. A voltage is generated between the electrodes, depending on the concentration of sulphur compounds in the liquid, which may therefore be determined.

US-2009/0317299 relates to an optical sensor to determine the sulphur level in a fuel. This is done by illuminating the fuel with a suitable wavelength spectrum, detecting the reflected light, and then analysing this in order to obtain a detection signal that specifies the sulphur level.

Both these prior art sensors are deemed to be active since they require some form of power supply in connection with the detection or when the signal processing is carried out. The measurements described in both these published patent applications provide a direct measurement result, i.e. a measurement value that reflects the current sulphur level.

A proposed alternative to an electrically powered sensor is described in SE 535895 C2, which shows an indication unit with a number of capsules or layers that absorb sulphur in contact with the fuel. The capsules contain liquid with different sulphur levels and the layers have differing uptakes of sulphur. At the analysis of the sulphur level in the respective capsules/layers, it is determined whether the level has increased and exceeds the original levels. If so, this is an indication that sulphur from the fuel has been supplied and that the sulphur level in the fuel has exceeded the predetermined level of the relevant capsule/layer.

US20090211379 discloses a device and method for sampling a fluid. An externally activated piston is used to regulate the pressure in the control chamber and thereby control check valves.

WO2013002713 discloses a device and method for sampling a fluid. A semipermeable membrane is used to allow liquid to be sampled into a sampling unit.

Despite prior art solutions, there is a need for easily detecting and/or analysing the occurrence and level of sulphur contaminants and other environmentally hazardous compounds in the fuel. There is also a need to collect information about the maximum level of a chemical compound, e.g. sulphur, in the fuel that the combustion engine has been exposed to, since a fuel with too high a sulphur level may deteriorate the function of the catalyst, which in turn means that the emission requirements for the exhausts may not be met. If the catalyst's function deteriorates so that the emission requirements are not complied with, and if the vehicle has not been fuelled with a fuel having a sulphur level exceeding e.g. 10 ppm, this may in the worst case entail that the vehicle manufacturer must recall and repair a large number of vehicles, which may be very costly. If on the other hand it can be proved that the instructions have not been complied with since fuel with more than e.g. 10 ppm has been used, such a campaign will not be necessary.

### SUMMARY OF THE INVENTION

Despite prior art solutions, there is a need further to develop sampling of a liquid, especially of a fuel, during a time period, which gives an indication, in an easy way, of the level of a substance over such time period. If the sampling unit is used in for example a vehicle, it is possible to analyse whether the vehicle has been fuelled with fuel exceeding predetermined levels for different chemicals or compounds. There is also a need for a sampling unit, which is passive and does not require any maintenance and therefore has a low cost.

The objective of the present invention is thus to provide a method for analysis of a liquid with a sampling unit, which in an easy way is adapted to collect a liquid sample, especially a fuel sample, for further analysis. The sample may then provide an indication as to whether the vehicle has been fuelled with fuel having chemical levels exceeding approved levels for the fuel, for example relating to sulphur level.

This objective is achieved with a method for analysis of a liquid with a sampling unit as defined in claim 1. Preferred embodiments are defined in the dependent claims.

The present invention uses a sampling unit for a liquid sample, preferably for a fuel intended for a combustion engine. The sampling unit is adapted to be fitted into a system with temperature variations, which system contains or transports a liquid. The sampling unit comprises a wall section partly surrounding a liquid filled cavity and a first opening, through which the liquid in the cavity may flow out of the cavity, and a second opening through which the liquid in the system may flow into the cavity. The first opening is equipped with a first shutter element, which is opened when the liquid's temperature in the cavity increases, the liquid expands and there is an over pressure in the cavity. The shutter element is closed when the pressure between the cavity and the system has equalised, i.e. there is no pressure difference between the system and the cavity. The second opening is equipped with a second shutter element, which opens when the liquid's temperature in the cavity drops and a negative pressure is created in the cavity. The second shutter element is closed when the pressure between the system and the cavity has equalised. Accordingly, the liquid flows out of the cavity when the liquid's temperature increases and the liquid expands, and the liquid flows into the cavity from the system when the liquid's temperature in the cavity drops and the liquid is compressed.

With such a sampling unit it is possible to continuously collect a liquid sample from a system during a time period, and subsequently to determine the level of a certain substance which the liquid has contained, as and when needed. The time period may be determined based on need. For example, if the DOC-unit's function has deteriorated, there is a need to analyse what fuel has been fuelled. The liquid in the sampling unit contains a mixture of liquids, which has passed through the sampling unit during the predetermined time period. The mixture of liquids may take place when the liquid in a liquid filled sampling unit is compressed as the temperature drops and a small amount of liquid is allowed to flow into the cavity, in which the liquids are mixed. This may occur since the second shutter element is opened because of the under pressure in the liquid inside the cavity and accordingly a small amount of liquid may flow into the cavity. When the temperature of the liquid increases, the liquid expands and the liquid flows out of the cavity. The first shutter element is then opened because of the pressure in the liquid and a small amount of liquid may flow out of the cavity to the system. The amount of liquid, which flows into the cavity of the sampling unit and out of the cavity depends on the sampling unit's design and temperature variations in the system. The compression and/or expansion of the liquid is proportionate to the temperature variation and may be determined experimentally and/or by way of calculations. Only a part of the liquid in the sampling unit is replaced at a time and the liquid in the sampling unit may be given a replacement time or turnover time, which may be determined based on the temperature variation. As only a part of the liquid inside the sampling unit is replaced at a time, one may thus obtain an indication of the liquid's composition during the sampling period and accordingly it is also possible to obtain an indication as to unlawful levels of a substance in the liquid.

The above objectives are achieved also by a sampling unit cluster comprising at least two sampling units described in general terms above.

When the sampling unit is used in a fuel system to collect a sample from a fuel, it is possible to obtain an indication of for example the sulphur level that a fuel has contained during, for example, a long time period. It is therefore possible, for example in connection with too high sulphur levels in the fuel, to obtain an indication of the reason why an exhaust purification device has been put out of service. In the event fuel with a sulphur level above suitable levels has been used and the exhaust purification device has been put out of service, the user may obtain information that (s)he should use fuel with the prescribed sulphur level in the future. In the event the user did not know about the fuel's high sulphur level, the user may make demands on the fuel supplier, who must specify the correct sulphur level of the fuel.

The above specified objectives are achieved also using a system subjected to temperature variations between a resting temperature and an operating temperature. The operating temperature may be higher than the resting temperature but the resting temperature may also be higher than the operating temperature. The system comprises a hollow component, which contains or transports a liquid. Preferably the system is a fuel system, which comprises a number of components, wherein at least one sampling unit, or a sampling unit cluster as described above, is fitted into at least one of the components.

The objectives specified above are also achieved using a combustion engine and a vehicle comprising the combustion engine with a fuel system having a sampling unit, or a sampling unit cluster as described above. As there are also different standards and statutory requirements governing permitted exhaust emissions from vehicles, it is of interest both to the vehicle manufacturer and the user of the vehicle that the exhaust purification device of the vehicle functions correctly. The sampling unit used in the invention provides both the vehicle manufacturer and the user with an indication as to whether the fuel powering the vehicle's combustion engine has had too high a level of a substance, chemical or compound, for example the sulphur level.

Through the method the level of a substance in the liquid may be analysed easily, for example the level of sulphur in a fuel.

Other features and advantages of the invention are set out in the example descriptions below.

### BRIEF DESCRIPTION OF THE DRAWINGS

Below is a description, as an example, of preferred embodiments of the invention with reference to the enclosed drawings, in which:
- Fig. 1: shows a schematic side view of a vehicle with a sampling unit,
- Fig. 2: shows an example of a coupling diagram for a fuel,
- Fig. 3a-3d: shows a schematic illustration of the sampling unit's function,
- Fig. 4a-4c: shows a schematic cross section view of the sampling unit cluster,
- Fig. 5: shows a cross section view of another embodiment of a sampling unit cluster, and
- Fig. 6a-6c: shows a schematic illustration of the sampling unit cluster's function.

### DETAILED DESCRIPTION

The invention is described below with reference to the method, which is described generally above.

The sampling unit used in the present invention is intended to collect a liquid sample. Preferably, the liquid is a fuel intended for a combustion engine, but the liquid may be another liquid, which may be used within the process industry. When the sampling unit is used, it is fitted into a component of a system, which is subjected to temperature variations. The sampling unit may thus be used in a fuel system or in another system within the process industry.

The sampling unit comprises a wall section partly surrounding a cavity, which is liquid filled, a first opening through which the liquid in the system may flow out of the cavity, and a second opening through which the liquid may flow into the cavity. The sampling unit is arranged to be in contact with a liquid, e.g. a fuel, which is to be analysed, as an exchange of liquids between the cavity and the surrounding hollow component may occur. The liquid sample may be collected continuously during a time period from the liquid, with which the sampling unit is in contact. The time period is determined based on the need for analysis.

The liquid inside the cavity is replaced because of the liquid's expansion and compression at temperature variations between a resting temperature and an operating temperature. The temperature variation between a resting temperature and an operating temperature may vary greatly, and may for example be between 5 to 40°C, for example if the sampling unit is used in the fuel system of a truck, but is not limited to such variation. The resting temperature may correspond to the average ambient temperature, e.g. when the truck engine is turned off. The operating temperature may be higher than the resting temperature, but may also be lower than the operating temperature. The temperature may vary somewhat also within the resting temperature area and the operating temperature area.

The samples are collected by the cavity first being filled with a starting liquid, which may be a "pure" liquid, e.g. fuel whose sulphur level is below 10 ppm. At a resting temperature, which may for example be approximately 20°C, the liquid in the cavity of the sampling unit is at a normal position, i.e. the cavity is liquid filled. At an operating state, for example at operation of a truck, the liquid's temperature increases inside the cavity so that the liquid expands. When the system is shut off, the temperature drops again to a normal temperature and the liquid is compressed. the sampling unit's first opening, which is equipped with a first shutter element, allows the liquid to flow out of the cavity when the liquid's temperature in the cavity rises. Accordingly, a small amount of liquid may flow out of the cavity. The second opening is equipped with a second shutter element, which allows the liquid to flow into the cavity when the liquid's temperature in the cavity drops. Accordingly, a small amount of liquid may flow into the cavity. In this manner, the liquid inside the cavity may be mixed with the liquid in the system a little at a time. The sampling device then obtains a "liquid memory", which is an average composition of the liquids over a time period where several cycles of temperature increases and subsequent temperature drops occur. As the exchange of the liquid in the sampling unit occurs relatively slowly, it is possible to check e.g. the average sulphur level over a longer period with the sampling unit. After the sampling period, the contents of the sampling unit are analysed with a suitable method according to prior art.

The sampling unit is especially suitable for collecting fuel samples, especially diesel samples, in a fuel system of a vehicle. The molecules in diesel comprise mainly hydrocarbons between 10 and 22 carbon atoms, e.g. alkanes, aromatics, naphthenes and olefins, but the fuel also contains sulphur compounds and other non-organic compounds such as phosphorous compounds.

The sampling unit's cavity is liquid filled and may receive the liquid sample. The cavity is partially surrounded by the wall section of the sampling unit. The sampling unit has a shape and a size, which is suitable for the liquid sample in question. For example, the cavity may have a drop shape, a conical shape, a cylinder shape or for example the shape of an elongated cylinder. With such a shape a small contact surface with the liquid may be obtained in relation to the cavity's volume. An elongated cylinder shape may be advantageous since such a sampling unit is easy to manufacture and to arrange in different systems. According to one embodiment the first opening may be placed at a first end of the sampling unit and the second opening may be placed at a second end of the sampling unit, facing the first opening. Accordingly, a better mixture of liquids inside the cavity may be obtained. The cavity may have a size or volume from for example approximately 1 to 30 cm³, for example 20 cm³, but is not limited to such volumes.

When the sampling unit is fitted into the component, which may occur for example through the component's wall, it may come into contact with the liquid inside the hollowness. The fitting may be carried out in a variety of ways. For example, the wall may be drilled so that a through hole is formed in the wall. Subsequently, the sampling unit is fitted and fastened in the holes with a suitable attaching device. The sampling unit may also be shaped as a screw with a hollowness constituting the cavity and the attachment may be carried out with the help of threads on the outside of the screw. The sampling unit may, however, be fitted into the component with the help of other attachment methods and may for example be placed inside a component and screwed on the inside of the component wall so that it may come into contact with the surrounding liquid.

The sampling unit is preferably detachably fitted into the hollow component. Accordingly, the liquid may easily be emptied from the cavity.

The first and the second shutter elements may consist of check valves. The shutter element or check valve, which comprises an opening element, has a specified activation pressure. The opening element may e.g. be a ball or cone, which is opened when the pressure, caused by the liquid's expansion, or the negative pressure caused by the liquid's compression, is equal to or greater than the activation pressure. The check may then be opened with a specified activation pressure and closed automatically when the pressure is smaller than the activation pressure. The term activation pressure means a pressure, which is required to open the check valve. The pressure is generated by the liquid when the liquid expands/compresses at temperature variations. When the pressure difference between the cavity and the system is greater than the activation pressure of the check valve, the opening element in the check valve opens.

The activation pressure is adapted so that the check valve opens when the temperature increases from a lower temperature, which may be a resting temperature, to a higher temperature, which may be an operating temperature, to allow the in- or out-flow of the liquid. The check valve is adapted to be closed at a stable temperature when no or only an insignificant temperature variation is the system occurs. Preferably the activation pressure is adapted so that the check valve remains closed at small temperature variations, e.g. from approximately 1 to 5°C, but the interval is not limited to this. In this manner, it is possible to prevent frequent exchange of the liquid inside the cavity at minor temperature variations in the system. Alternatively, the activation pressure may be adapted so that the check valve opens also at small temperature variations.

The opening element of the check valve may be equipped with a spring element, entailing a specified activation pressure. Accordingly, the check valve's opening element may be adapted to resist predetermined temperature variations, and a sampling unit that functions at high temperature variations, e.g. from approximately 10-100°C, may be obtained. When the activation pressure varies the liquid's exchange speed inside the cavity may also be varied. the higher the activation pressure, the slower the exchange speed that may be obtained for the sampling unit.

The invention also uses a sampling unit cluster, comprising at least two sampling units as described above. A sampling unit in the cluster comprises shutter elements or check valves with a specified activation pressure, which may be adapted to prevailing temperature variations in the system. The activation pressure may be different for the respective sampling units. In such a cluster, liquid samples may accumulate over a time period with sampling units having different exchange speeds. Accordingly, the level a chemical may be estimated over a certain period.

The sampling units in the cluster are preferably arranged in a series, but may also be arranged in parallel. When the cluster comprises two sampling units in a series, the first sampling unit's second opening may be in contact with the liquid in the system. The first sampling unit's first opening, through which the liquid may flow out of the cavity then constitutes the second sampling unit's second opening, through which the liquid may flow into the second sampling unit's cavity. In this case the second sampling unit's first opening is in contact with the liquid in the system and the liquid may flow out of the system through this opening. When the cluster comprises more than two sampling units, the previous sampling unit's first opening is arranged so that it constitutes the subsequent sampling units second opening. The last sampling unit in a series comprises a first opening, which is in contact with the liquid in the system, and the liquid from the cluster may flow out of this opening.

The disclosure also relates to a system, not falling under the scope of the claimed invention, which is subjected to temperature variations between a resting temperature and an operating temperature. The operating temperature is usually higher than the resting temperature, but may also be lower than the resting temperature. The system comprises a hollow component, which contains or transports a liquid. The system comprises a sampling unit according to the above fitted into the hollow component, or a sampling unit cluster, as described above, fitted into the hollow component.

Preferably the system is a fuel system comprising a fuel tank, a fuel conduit, a feeding pump, a fuel filter, a high pressure pump, an accumulator and an injection system. The high pressure pump, the accumulator and the injection system constitute components in the fuel system's high pressure system and the feeding pump and the fuel filter constitute components in the fuel system's low pressure system. The pressure in the high pressure system may be approximately 1800-2500 bar and the pressure in the low pressure system may be approximately 8-15 bar. At last one sampling unit described above is fitted into the fuel tank and/or in at least one of a fuel conduit, feeding pump or fuel filter, which are components of the fuel system.

The sampling unit or sampling unit cluster may be fitted into the fuel tank. Accordingly, the sampling unit or sampling unit cluster comes into contact with the fuel that has been loaded. The sampling unit or sampling unit cluster may also alternatively or as a complement be fitted into a component in the fuel system's low pressure system. The sampling unit or sampling unit cluster may for example be fitted into the main fuel filter. It is easy to replace and monitor the sampling unit or sampling unit cluster if it is fitted into the fuel system's low pressure system or in the fuel tank.

The disclosure also relates to a vehicle, not falling under the scope of the claimed invention, comprising the fuel system described above.

The invention relates to a method for analysis of a liquid with a sampling unit or with a sampling unit cluster as described above.

The operating temperature is higher than the resting temperature within the method.

Preferably the liquid is a fuel and the system is a fuel system and the substance to be analysed is sulphur.

In order to determine the sulphur level of the fuel the liquid must be analysed. This suitably occurs after the sampling unit has been removed from the fuel system. The sulphur level of the fuel may be analysed according to standard methods such as those described in Swedish Standard SS-EN ISO 20884 (Petroleum products - Determination of sulphur content in fuels - Wavelength-dispersive X-ray spectroscopy (ISO 20884:2011) and / or Swedish Standard SS-EN ISO 20846 (Petroleum products - Determination of sulphur content in fuels - Ultraviolet Fluorescence Method (ISO 20846:2011). From the analysis result an indication is obtained as to whether the tested fuel has a sulphur level, which is higher than the recommended level for the fuel, which is below 10 ppm.

Other advantages of the invention are set out in the description below with reference to the enclosed drawings.

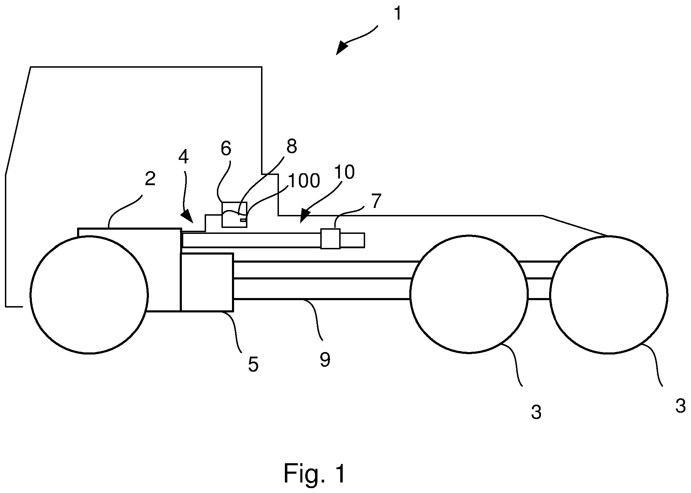

Figure 1 shows a vehicle 1 in a schematic side view, which vehicle 1 is equipped with a combustion engine 2, which operates the driving wheels 3 of the vehicle 1 via a gearbox 5 and a propeller shaft 9. The combustion engine 2 is equipped with an exhaust system 10. The combustion engine 2 is operated by a fuel 8, which is fed to the combustion engine 2 with a fuel system 4 comprising a fuel tank 6. The fuel system 4 also comprises a sampling unit 100 according to the disclosure, which in this example is placed in the fuel tank 100. The vehicle also comprises a chassis 7. The exhaust system 10 may comprise for example a diesel oxidation catalyst (DOC), a particulate filter and an EGR-system and an SCR-system.

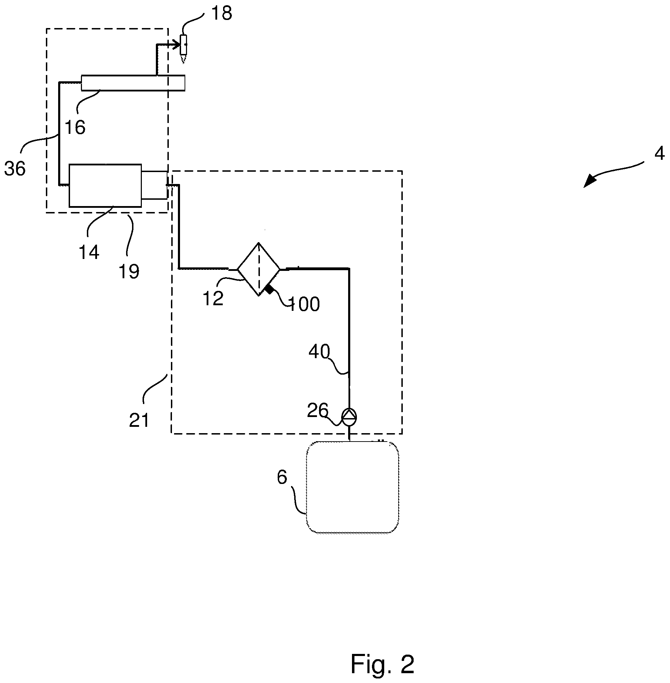

Fig. 2 shows an example of a coupling diagram for a fuel system 4 for a combustion engine 2.

The sampling unit 100 used in the present invention may be used for example in such a fuel system, but other versions of the fuel system may be in question. The sampling unit may also be used in other liquid systems, e.g. within the process industry.

The fuel system 4 comprises several components, of which a main fuel filter 12, a high pressure pump 14, an accumulator in the form of a so-called common rail 16, and an injection system 18 schematically displayed in the form of fuel injectors arranged in the combustion engine 2 (the combustion engine 2 is displayed in Fig. 1). Alternatively, the common rail 16 may be replaced by another form of an injection system 18, e.g. a piezo- or a unit injection system. The high pressure pump 14, the common rail 16 and the injection system 18 constitute components in the fuel system's 4 high pressure system 19. A sampling unit used in the present invention may be placed in any of the high pressure system's components, for example in a fuel conduit between the high pressure pump 14 and the common rail 16.

The fuel system 4 also comprises a fuel tank 6 and a feeding pump 26. These components may be arranged at the vehicle's chassis 7 (chassis 7 is displayed in Fig. 1). The fuel filter 12 is arranged downstream of the pump 26 and upstream of the high pressure pump 14 in the fuel system 4. The sampling unit 100 may be fitted into the fuel filter 12, as displayed in this example, but other placements are also possible, for example in a fuel conduit 40.

The feeding pump 26 pressurises the fuel in a low pressure system 21 of the fuel system and feeds the fuel from the fuel tank 20 via the fuel conduit 40 through the main fuel filter 12 and further along to the high pressure pump 14. The fuel is then fed, at a high pressure, to the common rail 16 and further along to the injection system 18.

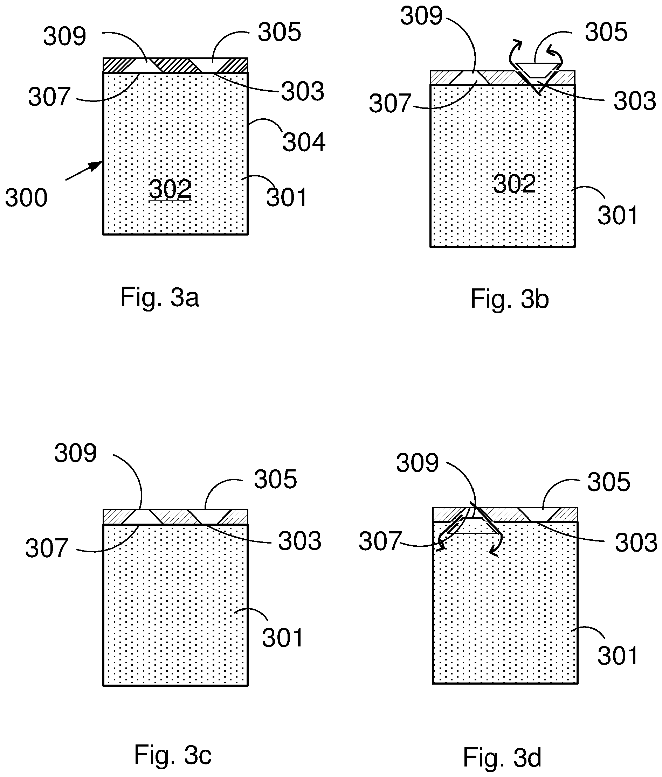

Fig. 3a-3d show schematically the function of a sampling unit 300. The sampling unit 300 comprises a wall section 304, which partly surrounds a cavity 301, a first opening 303 with a first check valve 305, and a second opening 307 with a second check valve 309. The sampling unit is filled with a starting fluid 302. The check valves 305 and 309 have a specified activation pressure and the first check valve 305 allows flow out from the cavity when the temperature of the liquid 302 in the cavity 301 increases and the second check valve 309 allows inflow into the cavity 301 when the temperature of the liquid in the cavity 301 drops. Fig. 3a illustrates a state at a normal temperature in a system, which may be e.g. at 20°C. In this state the liquid 302 fills the cavity 301. Fig. 3b illustrates a situation in the system where the temperature increases from a resting temperature to an operating temperature and the liquid's temperature in the cavity increases, e.g. between approximately 5-40°C. Because of the increased temperature of the liquid 302 in the cavity 301, the liquid 302 expands and the pressure in the liquid increases above the activation pressure of the first check valve 305, and a small amount of liquid 302 in the cavity 301 may flow out through the first opening 303 to a surrounding component in the system, for example a fuel tank, as shown by the arrows. Fig. 3c shows the situation after the liquid has been released from the cavity 301 and when the temperature has stabilised to the operating temperature and the pressure inside the cavity 301 has dropped. Both check valves 305 and 309 are closed and no liquid may flow in or out of the cavity 301. Fig. 3d shows the situation after the temperature in the cavity 301 drops from the operating temperature back to a normal temperature. As the liquid's temperature drops, the liquid 302 is compressed inside the cavity 301, so that a negative pressure is created inside the cavity 301. The negative pressure exceeds the activation pressure of the second check valve 309, the check valve opens and the liquid from the system may flow into the cavity 301 through the second opening 307. A small amount of liquid, which may correspond to the amount of liquid released from the cavity at expansion, flows into the cavity 301 from the surrounding component in the system. After a normal pressure in the cavity 301 has been achieved, the check valve 309 is closed and the sampling unit 300 returns to a normal state as displayed in Fig. 3a. Accordingly, the liquid inside the cavity 301 has been mixed with the surrounding liquid. During a sampling period several cycles of increased temperatures and subsequent temperature drops occur, and therefore a mixture of liquids may be obtained during a fixed period.

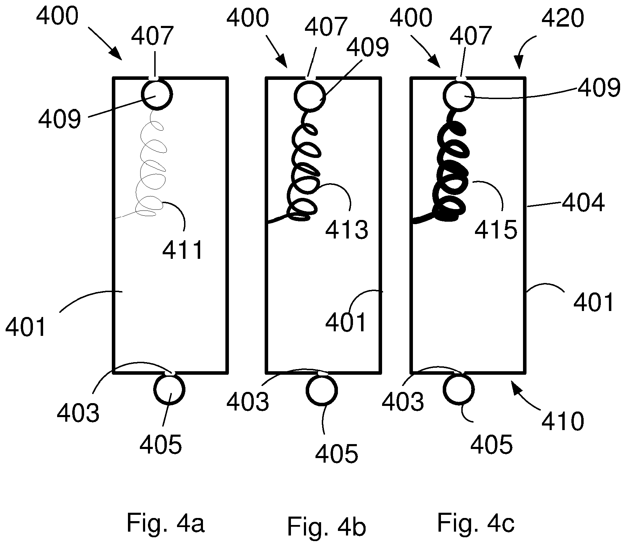

Fig. 4a-4c shows examples of a possible designs of a sampling unit used according to the invention. In each of the examples in Fig. 4a-4c the sampling unit 400 comprises a wall section 404 (displayed in Fig. 4c), which partly surrounds a cavity 401, a first opening 403, a second opening 407, a first check valve 405 and a second check valve 409. Each one of the second check valves 409 comprise a spring element in the form of a spring 411 (Fig. 4a); 413 (Fig. 4b); 415 (Fig. 4c). These springs have different stiffness and they impact the activation pressure of the second check valve 409. The spring 411 has the least stiffness and accordingly the lowest activation pressure and the spring 415 has the most stiffness and accordingly the highest activation pressure. Accordingly the sampling unit's check valve 409 may be opened and closed with the spring 411 at lower temperature variations than for example the check valve 409 with the spring 415. Accordingly the sampling unit with the spring 411 may have a greater exchange of liquid inside the cavity 401 at each cycle than the sampling unit with the spring 415. Additionally, the sampling unit with the spring 411 may have an exchange even at low temperature variations. The sampling units 400 may be placed in a liquid system as separate units or as a cluster. Because of varying activation pressure, different exchange speeds are obtained for the liquid in the respective sampling units. The sampling unit 400 is designed as an elongated cylinder and the first opening 403 is placed at a first end 410 (shown in Fig. 4c) of the sampling unit 400 and the second opening 407 is placed at a second end 420 (displayed in Fig. 4c) of the sampling unit 400, facing the first opening 403. The sampling unit 400 may be equipped with threads on the outside of the wall section 404 (not displayed) to facilitate the attachment of the sampling unit 400 inside a hollow component.

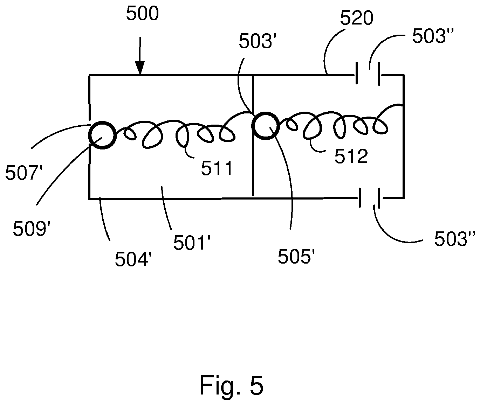

Fig. 5 another embodiment of a sampling unit 500 used according to the invention is shown, which sampling unit 500 comprises an outlet chamber 520. The sampling unit comprises a wall section 504', which partly surrounds a cavity 501'. The sampling unit comprises a first opening 503', which is equipped with a first check valve 505', through which the liquid may flow out of the cavity 501' to the outlet chamber 520. The outlet chamber receives the liquid from the sampling unit 500, but its task is also to facilitate attachment of a spring 512 in the check valve 505'. The sampling unit 500 also comprises a second opening 507', which is in contact with the liquid to be analysed in the system. The second opening 507' is equipped with a second check valve 509', through which the liquid may flow into the cavity 501'. The liquid flows out of the cavity 501' through the first opening 503' equipped with the first check valve 505' when the liquid's temperature in the cavity 501' increases, the liquid expands and an over-pressure is created in the cavity 501'. The liquid flows out of the cavity 501' to the outlet chamber 520, which is equipped with two openings 503", through which the liquid may flow freely in or out. When the liquid's temperature in the cavity 501' drops, a negative pressure is created in the cavity 501', so that the second check valve 509' opens and the liquid from the system may flow into the cavity 501' through the second opening 507'. The second check valve 509' is closed when the pressure between the cavity 501' and the system has equalised.

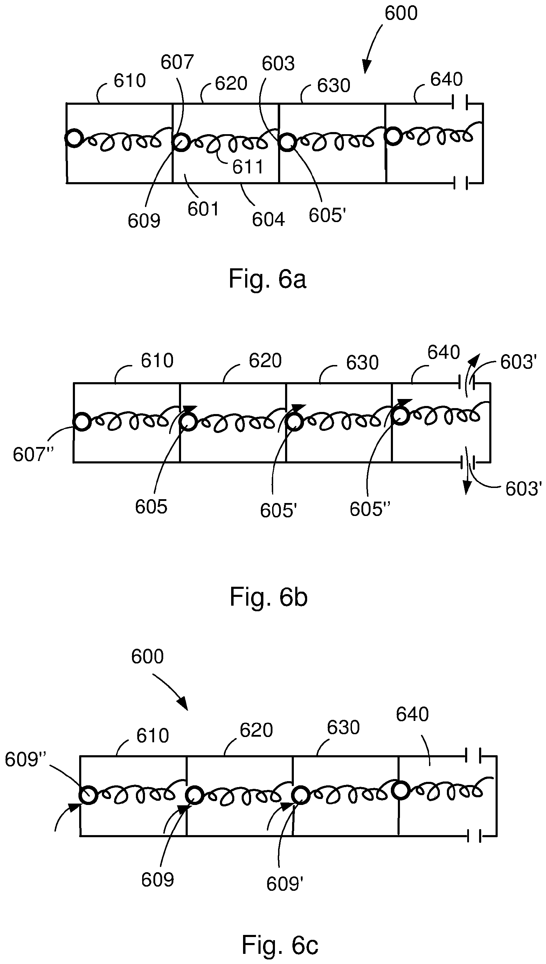

Fig. 6a shows a sampling unit cluster 600 comprising three sampling units 610, 620 and 630, and one outlet chamber 640, which are connected in series. The sampling units 610, 620 and 630 correspond to the sampling unit 500 in Fig. 5, and the outlet chamber 640 corresponds to the outlet chamber 520 in Fig. 5. In order to increase clarity in the drawings, the components of the sampling units 610, 620 and 630 are shown only with reference to the sampling unit 620. The sampling unit 620 comprises a wall section 604, which partly surrounds a cavity 601, a first opening 603, through which the liquid may flow out of the cavity 601 when the temperature increases e.g. from a resting temperature to an operating temperature and the liquid expands, and a second opening 607, through which the liquid may flow into the cavity 601 when the temperature drops and the liquid is compressed. Each one of the openings 603, 607 are equipped with a check valve 605', 609, which open when the pressure difference at the liquid's expansion or compression is greater than the activation pressure of the check valves 605', 609. The first check valve 605' is equipped with a spring 612, which is attached to the subsequent sampling unit 630 and the second check valve 609 is equipped with a spring 611, which is attached to the sampling unit 620. Fig. 6a shows all units 610, 620 and 630 filled with a starting fluid and the system in a resting state.

Fig. 6b shows the function of the cluster 600 when the temperature increases e.g. from the resting temperature to the operating temperature, the liquid expands and an over-pressure is created in the sampling units 610, 620 and 630. Each one of the sampling units 610, 620 and 630 comprises a first check valve 605, 605' and 605". The first check valves 605, 605' and 605" open and the liquid from the first sampling unit 610 flows into the second sampling unit 620, and similarly the liquid flows from the second sampling unit 620 to the third sampling unit 630. The liquid from the third sampling unit 630 flows into the outlet chamber 640, which is equipped with two openings 603', through which the liquid may flow freely in or out of the system. The system may be e.g. a fuel tank, in which the sampling unit may be fitted or loose, so that a second opening 609" in the sampling unit 610 and the openings 603' may come into contact with fuel in the tank. After the expansion the system reverts to a stable state as displayed in Fig. 6a.

Fig. 6c shows the function of the cluster when the temperature drops from the operating temperature to the resting temperature, the liquid is compressed and a negative pressure is created in the sampling units 610, 620 and 630. Each one of the sampling units 610, 620 and 630 comprises a second check valve 609", 609 and 609'. The second check valves 609", 609 and 609' are opened and the liquid from the system may flow into the first sampling unit 610 and similarly the liquid flows from the first sampling unit 610 to the second sampling unit 620 and from the second sampling unit 620 to the third sampling unit 630. The second check valves are closed after the pressure difference between the systems and the sampling units have been equalised. The first check valve 605" is closed and no liquid flows into the outlet chamber 640. Accordingly, the liquids in the sampling units may be mixed when several cycles of temperature variations occur during the sampling period. With such a cluster of sampling units, a slower exchange or turnover of the liquid may be achieved inside the cavity of the sampling units following the first sampling unit so that the exchange speed is slowest in the sampling unit, which is last in a series.

The foregoing description of the preferred embodiments of the present invention has been furnished for illustrative and descriptive purposes. The described embodiments are not intended to be exhaustive or to limit the invention, but the invention is limited by the scope of the enclosed claims.

## Claims

1. Method for analysis of a liquid with a sampling unit (100; 300; 400; 500; 610; 620; 630) for a liquid sample, preferably for a fuel intended for a combustion engine (2), which sampling unit (100; 300; 400; 500; 610; 620; 630) is adapted to be fitted into a system which contains or transports a liquid, wherein the sampling unit (100; 300; 400; 500; 610; 620; 630) comprises a wall section (304; 404; 504'; 604) partly surrounding a cavity (301; 401; 501'; 601), which is adapted to be filled with the liquid, a first opening (303; 403; 503'; 603), through which liquid in the cavity may flow out of the cavity and a second opening (307; 407; 507'; 507"; 607) through which liquid may flow into the cavity (301; 401; 501'; 601), wherein the first opening (303; 403; 503'; 603) is equipped with a first shutter element (305; 405; 505'; 605; 605'; 605"), which opens when the liquid's temperature in the cavity increases and the liquid expands and an over-pressure is created in the cavity, and wherein the second opening (307; 407; 507'; 607) is equipped with a second shutter element (309; 409; 509'; 609; 609'; 609"), which opens when the liquid's temperature in the cavity drops and a negative pressure is created in the cavity, **characterised in that** the method comprises the steps:
a) of filling the cavity (301; 401; 501'; 601) of the sampling unit (100; 300; 400; 500; 610; 620; 630) initially with a liquid;
b) of placing or fitting the sampling unit in a system, in which the sampling unit (100; 300; 400; 500; 610; 620; 630) comes into contact with a liquid to be analysed;
c) of subjecting the liquid in the system to temperature variations between a resting temperature and an operating temperature and vice versa;
d) of allowing the liquid in the cavity (301; 401; 501'; 601) to flow out of the cavity, using the first shutter element (305; 405; 505'; 605; 605'; 605"), when the temperature of the liquid in the cavity (301; 401; 501'; 601) increases and the liquid expands and an over-pressure is created in the cavity (301; 401; 501'; 601);
e) of allowing the liquid in the system to flow into the cavity (301; 401; 501'; 601), using the second shutter element (309; 409; 509'; 609; 609'; 609"), when the liquid's temperature in the cavity (301; 401; 501'; 601) drops and a negative pressure is created in the cavity (301; 401; 501'; 601); and
f) of allowing mixture of the liquid inside the cavity (301; 401; 501'; 601) by way of the liquid's in- and out-flow.

2. Method according to claim 1, **characterised in that** the method further comprises the steps:
g) of removing the sampling unit (100; 300; 400; 500; 610; 620; 630) from the system after a time period,
h) removing the liquid accumulated in the sampling unit (100; 300; 400; 500; 610; 620; 630), and
i) of analysing the level of a substance in the liquid from the sampling unit (100; 300; 400; 500; 610; 620; 630) with an analysis method adapted to the substance to be analysed.

3. Method according to claim 1 or 2, **characterised in that** the liquid is a fuel and the system is a fuel system (4).

4. Method according to claim 3, **characterised in that** the substance to be analysed is sulphur.

5. Method according to any one of the preceding claims, **characterised in that** the method is performed with a sampling unit cluster (600) comprising at least two sampling units (610; 620; 630) arranged in a series.

## Patentansprüche

1. Verfahren zur Analyse einer Flüssigkeit mit einer Probenahmeeinheit (100; 300; 400; 500; 610; 620; 630) für eine flüssige Probe, vorzugsweise für einen für einen Verbrennungsmotor (2) vorgesehenen Kraftstoff, wobei die Probenahmeeinheit (100; 300; 400; 500; 610; 620; 630) dazu eingerichtet ist, in ein System eingebaut zu werden, das eine Flüssigkeit enthält oder transportiert, wobei die Probenahmeeinheit (100; 300; 400; 500; 610; 620; 630) einen Wandabschnitt (304; 404; 504'; 604), der einen Hohlraum (301; 401; 501'; 601) teilweise umgibt, welcher dazu eingerichtet ist, mit der Flüssigkeit befüllt zu werden, eine erste Öffnung (303; 403; 503'; 603), durch die Flüssigkeit in dem Hohlraum aus dem Hohlraum herausfließen kann, und eine zweite Öffnung (307; 407; 507'; 507"; 607) aufweist, durch die Flüssigkeit in den Hohlraum (301; 401; 501'; 601) fließen kann, wobei die erste Öffnung (303; 403; 503'; 603) mit einem ersten Verschlusselement (305; 405; 505'; 605; 605'; 605") versehen ist, das sich öffnet, wenn die Temperatur der Flüssigkeit in dem Hohlraum ansteigt und die Flüssigkeit sich ausdehnt und ein Überdruck in dem Hohlraum entsteht, und wobei die zweite Öffnung (307; 407; 507'; 607) mit einem zweiten Verschlusselement (309; 409; 509'; 609; 609'; 609") versehen ist, das sich öffnet, wenn die Temperatur der Flüssigkeit in dem Hohlraum fällt und ein Unterdruck in dem Hohlraum entsteht, **dadurch gekennzeichnet, dass** das Verfahren die Schritte umfasst:
a) anfängliches Befüllen des Hohlraums (301; 401; 501'; 601) der Probenahmeeinheit (100; 300; 400; 500; 610; 620; 630) mit einer Flüssigkeit,
b) Platzieren oder Einbauen der Probenahmeeinheit in ein System, in dem die Probenahmeeinheit (100; 300; 400; 500; 610; 620; 630) in Kontakt mit einer zu analysierenden Flüssigkeit kommt,
c) Aussetzen der Flüssigkeit in dem System Temperaturschwankungen zwischen einer Ruhetemperatur und einer Betriebstemperatur und umgekehrt,
d) Gestatten der Flüssigkeit in dem Hohlraum (301; 401; 501'; 601), unter Benutzung des ersten Verschlusselements (305; 405; 505'; 605; 605'; 605") aus dem Hohlraum herauszufließen, wenn die Temperatur der Flüssigkeit in dem Hohlraum (301; 401; 501'; 601) ansteigt und die Flüssigkeit sich ausdehnt und ein Überdruck in dem Hohlraum (301; 401; 501'; 601) entsteht,
e) Gestatten der Flüssigkeit in dem System, unter Benutzung des zweiten Verschlusselements (309; 409; 509'; 609; 609'; 609") in den Hohlraum (301; 401; 501'; 601) zu fließen, wenn die Temperatur der Flüssigkeit in dem Hohlraum (301; 401; 501'; 601) fällt und ein Unterdruck in dem Hohlraum (301; 401; 501'; 601) entsteht, und f) Gestatten einer Vermischung der Flüssigkeit innerhalb des Hohlraums (301; 401; 501'; 601) mittels des Ein- und Ausströmens der Flüssigkeit.

2. Verfahren nach Anspruch 1, **dadurch gekennzeichnet, dass** das Verfahren ferner den Schritt umfasst:
g) Entfernen der Probenahmeeinheit (100; 300; 400; 500; 610; 620; 630) aus dem System nach einer Zeitspanne,
h) Entnehmen der in der Probenahmeeinheit (100; 300; 400; 500; 610; 620; 630) akkumulierten Flüssigkeit, und
i) Analysieren des Gehalts einer Substanz in der Flüssigkeit aus der Probenahmeeinheit (100; 300; 400; 500; 610; 620; 630) mit einem Analyseverfahren, das an die zu analysierende Substanz angepasst ist.

3. Verfahren nach Anspruch 1 oder 2, **dadurch gekennzeichnet, dass** die Flüssigkeit ein Kraftstoff ist und das System ein Kraftstoffsystem (4) ist.

4. Verfahren nach Anspruch 3, **dadurch gekennzeichnet, dass** die zu analysierende Substanz Schwefel ist.

5. Verfahren nach einem der vorhergehenden Ansprüche, **dadurch gekennzeichnet, dass** das Verfahren mit einem Probenahmeeinheit-Cluster (600) ausgeführt wird, der wenigstens zwei in Reihe angeordnete Probenahmeeinheiten (610; 620; 630) umfasst.

## Revendications

1. Procédé d'analyse d'un liquide avec une unité d'échantillonnage (100 ; 300 ; 400 ; 500 ; 610 ; 620 ; 630) pour un échantillon liquide, de préférence pour un carburant destiné à un moteur à combustion (2), qui est l'unité d'échantillonnage (100 ; 300 ; 400 ; 500 ; 610 ; 620 ; 630) est adapté pour être monté dans un système qui contient ou transporte un liquide, dans lequel l'unité d'échantillonnage (100 ; 300 ; 400 ; 500 ; 610 ; 620 ; 630) comprend une section de paroi (304 ; 404 ; 504' ; 604) entourant partiellement une cavité (301 ; 401 ; 501' ; 601), qui est adapté pour être rempli avec le liquide, une première ouverture (303 ; 403 ; 503' ; 603), à travers lequel le liquide dans la cavité peut s'écouler hors de la cavité et une seconde ouverture (307 ; 407 ; 507' ; 507" ; 607) à travers laquelle le liquide peut s'écouler dans la cavité (301 ; 401 ; 501' ; 601), dans lequel la première ouverture (303 ; 403 ; 503' ; 603) est équipé d'un premier élément d'obturation (305 ; 405 ; 505' ; 605 ; 605' ; 605"), qui s'ouvre lorsque la température du liquide dans la cavité diminue et que le liquide se dilate et une surpression est créée dans la cavité, et dans lequel la seconde ouverture (307 ; 407 ; 507' ; 607) est équipé d'un deuxième élément d'obturation (309 ; 409 ; 509' ; 609 ; 609' ; 609"), qui s'ouvre lorsque la température du liquide dans la cavité chute et qu'une pression négative est créée dans la cavité, **caractérisé en ce que** le procédé comprend les étapes :
a) du remplissage de la cavité (301 ; 401 ; 501' ; 601) de l'unité d'échantillonnage (100 ; 300 ; 400 ; 500 ; 610 ; 620 ; 630) initialement avec un liquide ;
b) du placement ou du montage de l'unité d'échantillonnage dans un système, dans lequel l'unité d'échantillonnage (100 ; 300 ; 400 ; 500 ; 610 ; 620 ; 630) entre en contact avec un liquide à analyser ;
c) de soumission du liquide dans le système à des variations de température entre une température de repos et une température de fonctionnement et vice versa ;
d) de l'écoulement du liquide dans la cavité (301 ; 401 ; 501' ; 601) hors de la cavité, en utilisant le premier élément d'obturation (305 ; 405 ; 505' ; 605 ; 605' ; 605"), lorsque la température du liquide dans la cavité (301 ; 401 ; 501' ; 601) augmente et que le liquide se dilate et qu'une surpression est créée dans la cavité (301 ; 401 ; 501' ; 601) ;
e) de l'écoulement du liquide dans la cavité (301 ; 401 ; 501' ; 601), en utilisant le second élément d'obturation (309 ; 409 ; 509' ; 609 ; 609' ; 609"), lorsque la température du liquide dans la cavité (301 ; 401 ; 501' ; 601) chute et qu'une pression négative est créée dans la cavité (301 ; 401 ; 501' ; 601), et
f) de possibilité du mélange du liquide à l'intérieur de la cavité (301; 401; 501'; 601) au moyen du flux d'entrée et de sortie du liquide ;

2. Procédé selon la revendication 1, **caractérisé en ce que** le procédé comprend en outre les étapes :
g) du retrait de l'unité d'échantillonnage (100 ; 300 ; 400 ; 500 ; 610 ; 620 ; 630) du système après une période de temps ;
h) du retrait du liquide accumulé dans l'unité d'échantillonnage (100 ; 300 ; 400 ; 500 ; 610 ; 620 ; 630), et
i) d'analyse du niveau d'une substance dans le liquide à partir de l'unité d'échantillonnage (100 ; 300 ; 400 ; 500 ; 610 ; 620 ; 630) ou du groupe d'unités d'échantillonnage avec un procédé d'analyse adapté à la substance à analyser.

3. Procédé selon la revendication 1 ou 2, **caractérisé en ce que** le liquide est un carburant et le système est un système de carburant (4).

4. Procédé selon la revendication 3, **caractérisé en ce que** la substance à analyser est du soufre.

5. Procédé selon l'une quelconque des revendications précédentes, **caractérisé en ce que** le procédé est réalisé avec un groupe d'unités d'échantillonnage (600) comprenant au moins deux unités d'échantillonnage (610 ; 620 ; 630) disposées en série.
